# EUROPEAN PATENT SPECIFICATION

(11) **EP 3 266 306 B1**
(45) Date of publication and mention of the grant of the patent: **03.08.2022**
(21) Application number: 17176211.5
(22) Date of filing: 15.06.2017
(51) Int. Cl.: A01P 1/00, A61L 2/16, A01N 25/10, A01N 25/34, A61K 8/34, A61Q 17/00, C11D 17/04, A01N 31/02

(54) **IMPREGNATED TEXTILE FABRIC PROVIDING EFFECTIVE DELIVERY OF AN ALCOHOLIC IMPREGNANT**
IMPRÄGNIERTES TEXTILGEWEBE ZUR BEREITSTELLUNG EINER WIRKSAMEN VERABREICHUNG EINES ALKOHOLISCHEN IMPRÄGNIERMITTELS
TISSU TEXTILE IMPRÉGNÉ ASSURANT L'APPORT EFFICACE D'UN AGENT D'IMPRÉGNATION ALCOOLISÉ

(30) Priority: 04.07.2016 DE 102016112163
(43) Date of publication of application: 10.01.2018
(73) Proprietor: SCHÜLKE & MAYR GmbH, 22851 Norderstedt (DE)
(72) Inventor: HARTMANN, Claas-Florian, 24641 SIEVERSHÜTTEN (DE); HEIDEL, Judith, 25421 PINNEBERG (DE); STEINHAUER, Katrin, 22459 HAMBURG (DE); TEUTENBERG, Lars, 22415 HAMBURG (DE); TECKEMEYER, Karin, 22415 HAMBURG (DE)
(74) Representative: LKGLOBAL Lorenz & Kopf PartG mbB Patentanwälte

(56) References cited:
- EP-A1- 2 886 637
- EP-A2- 1 281 393
- EP-B1- 0 398 891
- EP-B1- 1 128 725
- WO-A1-03/050224
- WO-A1-03/053144
- WO-A1-2013/102455
- DE-A1- 10 047 759
- Anonymous: "sawatex 2611/2011", Sandler AG, 1 January 2011 (2011-01-01), page 1pp, XP055707843,
- Paul Hartmann: "Alkoholische Schnelldesinfektion von Flachen", HARTMANN - Produktblatt, 1 February 2014 (2014-02-01), pages 1-16, XP055847586, Retrieved from the Internet: URL:https://hygiene.stangl.at/upload/PDFs/ 454.pdf [retrieved on 2021-10-05]
- MATHIAS SCHWEINS ET AL: "Einflussfaktoren auf die Flächenleistung wirkstoffgetränkter Einmal-Wischtücher zur Reinigung und Desinfektion im medizinischen Bereich", HYGMED, vol. 40, no. 4, 1 January 2015 (2015-01-01), pages 144-149, XP055703431,
- DR. ROLAND KNIELER: "Qualität und Prüfung von gebrauchsfertigen Desinfektionstüchern", HYGIENE & MEDIZIN, vol. 44, no. 4, 1 January 2019 (2019-01-01), pages D33-D40, XP055703437,

## Description

The invention relates to an impregnated textile fabric comprising a carrier material impregnated with an alcoholic preparation. The carrier material aside, the alcoholic preparation comprises one or more aliphatic C₂ to C₃ alcohols. The impregnated textile fabric is suitable in particular for use on animate surfaces (such as skin) or inanimate surfaces (as for disinfection of equipment and areas). The invention additionally provides a kit comprising a carrier material and an alcoholic preparation for use in the manufacture of such impregnated textile fabrics. The invention further provides a process for disinfecting animate or inanimate surfaces wherein the surface is subjected to the action of the impregnated textile fabric, and also to the use of the kit or of the impregnated fabric to disinfect animate and inanimate surfaces.

Moist cloths for use on animate or inanimate surfaces are known. DE 10 2010 049 113 A1 for instance discloses a textile fabric impregnated with an antimicrobial preparation and comprising a carrier material based on polyolefin. The antimicrobially active preparation comprises a bispyridinioalkane. The impregnated fabric is suitable in particular for use on animate surfaces, for example on human skin.

DE 10 2013 226 787 A1 relates to a textile fabric impregnated with an active antimicrobial and/or cleaning preparation and having a carrier comprising a bicomponent-fibre web comprising polyolefin. The fabric is suitable in particular for single use on inanimate hard surfaces, for example to disinfect or clean such surfaces. Specific carrier materials are stressed there to be advantageous for use in the disinfection and cleaning of a typical floor area (of about 20 m²) in a hospital because the entire amount of the active preparation is released without entailing any loss of active ingredient. Basis weights for the webs preferred there are typically in the range from 70 to 90 g/m². Active preparations having a high content of aliphatic alcohol are not addressed in 10 2013 226 787 A1.

WO 2013/102455 A1 discloses a liquid-impregnated specific nonwoven formed of bicomponent fibres. Again, impregnants having a content of aliphatic alcohol are not addressed in WO2013/102455.

DE 600 13 053 T2 (EP 1 086 648 B1) discloses pre-wetted wipes comprising not less than 1 g gram of liquid composition per gram of dry fibrous tissue, preferably not less than about 1.5 and particularly about 2 grams of liquid composition per gram of dry fibrous tissue. Pre-wetted wipes for cleaning walls are reported by DE 600 13 053 T2 to have a capacity of 2 to about 10 g per gram of dry fibrous tissue (that is, an absorbency of 200% to 1000%). Aqueous solventborne systems disclosed may include up to 5% of a solvent, such as ethanol or 2-propanol.

EP-B-1128725 discloses premoistened wipes comprising a nonwoven fibrous substrate including from 0.5 grams to 8 grams of liquid per gram of dry fibre, whereby the substrate also includes from 0.004% to 10% antimicrobial protease inhibitor by weight of said dry fibre. The dry, fibrous substrate can have a grammage of between about 40 to 100 grams per square meter. The substrate may include various combinations of cellulosic fibers, synthetic polymeric fibers such as polyester, polypropylene, polyethylene, and the like. When added to the liquid, the antimicrobial protease inhibitor comprises about 0.0005% to 10% of the liquid by weight. The liquid may comprise about 50% of solvent selected from the group consisting of oil, alcohol, water, and mixtures thereof.

EP-B-0398892 discloses disposable impregnated wipes for cleaning and maintaining surfaces, which consist of a woven or nonwoven hydrophilic material and which are obtained by wet or dry method. Said wipes have a maximum absorption capacity for water of at least 200 g by weight in the dry state and are impregnated with an aqueous composition to a level not exceeding 50% of the maximum absorption capacity. The aqueous composition may contain 5 to 40 wt% of the total weight of the aqueous solution watermiscible solvent selected from alcohols containing 1 to 4 carbon atoms, glycols and glycol ethers containing 2 to 8 carbon atoms, and volatile silicones.

DE-A-10047759 discloses body deodorization articles which consist of a flexible, absorbent, flat substrate which is impregnated with a liquid, deodorant preparation and enclosed in a liquid-tight packaging. The substrate may be a non-woven made of cellulose of viscose fibers or a mixture of cellulose fibers and fully synthetic fibers. The nonwoven preferably has a grammage of 20-100 g/m² and a capacity for absorbing water without spontaneous driping of at least 200%, preferably of more than 300%.

Spunlaced nonwovens impregnated with alcoholic gel for use as sanitary articles are also disclosed in EP-A-1281393. WO-A-2003/050224 discloses cleaning wipes which comprise 20 wt. % to 30 wt. % of a nonwoven fabric and (b) 70 wt. % to 80 wt. % of a liquid cleaning composition being impregnated in said nonwoven fabric. The liquid cleaning composition comprises 0.5 wt. % to 10 wt. % of a C1-C4 alkanol.

WO-A-2013/102455 discloses nonwovens with a grammage of 22 or 35 to about 50 g/m², which are impregnated with a disinfecting solution.

EP-A-2886637 discloses impregnated textile fabrics which comprise a carrier with a dry weight of 8 to 40 g, which comprises a bicomponent fibre fleece and an antimicrobial and/or cleaning active ingredient preparation with which the carrier is impregnated.

WO-A-2003/053144 discloses wipers comprising a substrate and a sanitizing formulation applied to said substrate in an amount from about 150% to about 600% of the dry weight of the wiper. The sanitizing formulation comprises water and between about 0.01 % by weight to about 1 % by weight of an antimicrobial agent. The sanitizing formulation may comprise between about 0.001% to about 30% by weight of non-aqueous solvent, such as glycols, alcohols, or combinations thereof. In one embodiment, the non- aqueous solvent includes a blend of ethanol and propylene glycol.

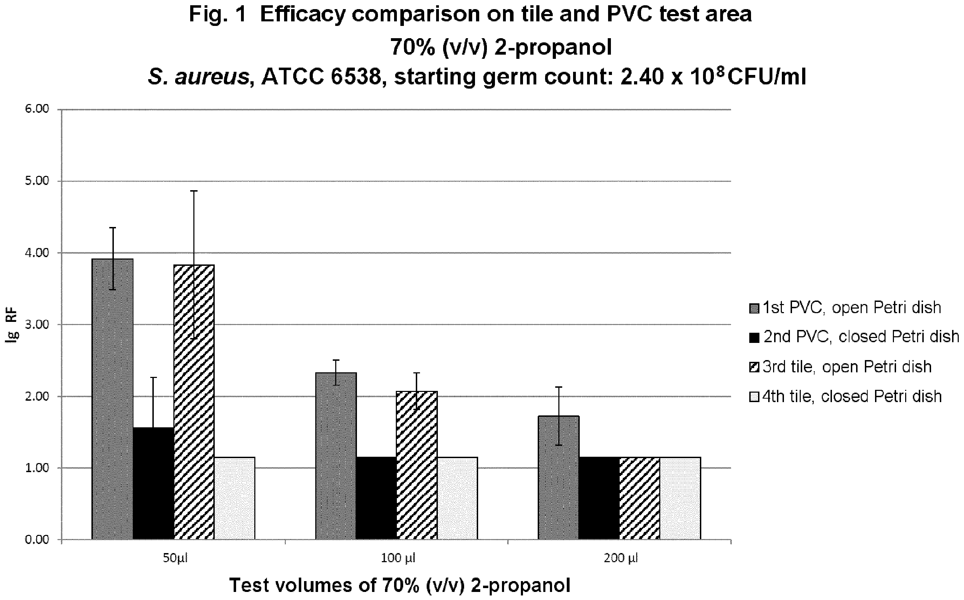

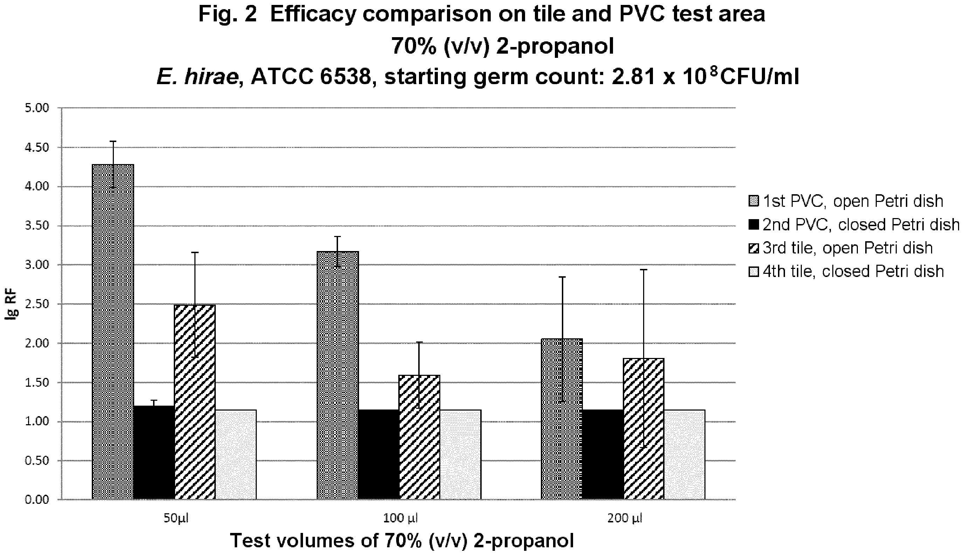

When alcoholic solutions are applied to an area, evaporation of the C₂ to C₃ alcohol used has an adverse impact on microbicidal efficacy. Tests have shown that microbicidal efficacy on the area in fact decreases significantly if the alcohol is able to evaporate (see Fig. 1 and Fig. 2). There is thus a direct correlation between the amount of active ingredient available on the area and the microbicidal efficacy.

Impregnating solutions for moist cloths have hitherto been tested for efficacy using a procedure described in Gebel, J., Werner, H.-P., Kirsch-Altena, A., Bansemir, K. (2001): [in German] Standard Methods of the German Society for Hygiene and Microbiology (DGHM) for Testing Chemical Disinfection Procedures, Method 14: Area Disinfection - A Realistic Test, pp. 40-44, mhp Verlag Wiesbaden. In this test, the efficacy of the disinfectants on artificially contaminated surfaces (OP tiles) is evaluated. The employment of mechanical action is simulated therein by using a spatula.

To make testing even more realistic, a new method for evaluating area disinfectants was developed to simulate their application via a standardized method of wiping defined PVC surfaces (EN 16615:2015; German Disinfectant Commission in the Association (VAH) for Applied Hygiene (2015): Requirements and Methods for VAH Certification of Chemical Disinfection Procedures, chapter 14.2 - Area Disinfection with Mechanical Action - A Realistic Test (4-field test), mhp Verlag Wiesbaden). The realistic EN 16615:2015 test accordingly requires for an adequate microbicidal efficacy that an effective amount of C₂ to C₃ alcohol be provided on the area to achieve microbicidal efficacy.

The present invention accordingly had for its object to provide cloths having a content of C₂ to C₃ alcohols and corresponding kits of carrier material and impregnating solution which are efficacious in the realistic test of EN 16615:2015, i.e. which in particular provide on the area an effective amount of C₂ to C₃ alcohol to achieve adequate microbicidal efficacy as per EN 16615:2015.

It was found that, surprisingly, the problems of the prior art are solved by a textile fabric impregnated with an alcoholic preparation, said fabric comprising
a) a carrier material, and
b) the alcoholic preparation,

wherein
   - the carrier material
      i) comprises one or more nonwovens selected from drylaid, airlaid, wetlaid and spunbonded nonwovens,
      ii) has a basis weight, determined as per DIN EN 29073-1, of 10 to 70 g/m², and
      iii) has an absorbency for the alcoholic preparation, determined in accordance with DIN 53923-1 of not less than 450% (wt./wt.), and
      iv) comprises one or more polymers selected from polyethylene terephthalate (PET), polyolefin, viscose, cellulose and polylactic acid (PLA),
   - the percentage impregnation of the carrier material with the alcoholic solution (the degree of impregnation) is not more than 80%,
and wherein
   - the alcoholic preparation comprises b1) two or more aliphatic C₂- to C₃ alcohols, wherein the total amount of alcohol component b1) is in the range from 20 to 80 wt%, based on the weight of alcoholic preparation b).

The present invention is based on the finding that an effective amount of C₂ to C₃ alcohol to achieve an adequate microbicidal efficacy as per EN 16615:2015 can be provided on the area from cloths.

The provision of alcoholic solution from the impregnated textile fabric is ensured by carrier materials having a high uptake capacity for the alcoholic preparation being impregnated, not to their maximum uptake capacity, but only up to a moderate degree of impregnation. This was surprising because a person skilled in the art would actually suppose that instead delivery would be particularly good at a very high degree of impregnation. It was further surprising that a particularly effective delivery is not achieved by using impregnatable materials having a high basis weight of more than 70 g/m², but that instead a moderate basis weight is particularly advantageous, provided the maximum uptake capacity of the carrier material is high.

The delivered quantity of alcoholic solutions out of cloths is thus significantly improvable by impregnating the nonwoven not, as is frequently customary, maximally, but only partially (to, for example, 50% of the absorbency). The invention thus provides the optimized release from cloths of solutions having a content of C₂ to C₃ alcohols. The evaporation effects of alcohols (see Figures 1 and 2) are controlled in the process as a result of the optimized release from the cloths.

Particularly the group of active ingredients which are alcohols requires an adequate quantity of delivery in order to be able to offset the evaporation effects observed and to achieve a high level of microbicidal efficacy. This is achieved according to the present invention by exploiting the surprising effect thereof regarding the ratio between delivery and uptake to impregnate nonwovens having a high uptake capacity moderately only, while nonetheless making possible a high quantitative delivery of volatile actives to the surfaces to be disinfected (see Table 3).

In the description of the present invention, the term "basis weight" (or "grammage") designates the value, reported in g/m², determined according to DIN EN 29073-1.

The term "absorbency" (or "maximum uptake") designates the maximum amount of the alcoholic preparation that can be taken up by the carrier material, based on the weight of the sheet material (i.e. maximum weight of alcoholic preparation that can be taken up by the carrier material/weight of the carrier material), reported in per cent. The value is determined in accordance with DIN 53923-1, as described in Method 1 in the examples.

The term "percentage impregnation of the carrier material" (or "degree of impregnation") is the quotient formed by dividing the absorbency into the amount of alcoholic preparation and reported in per cent. A 100% degree of impregnation is thus to be understood as meaning that the carrier material has been impregnated to its maximum absorbency.

### a) Carrier material

The carrier material i) comprises one or more nonwovens selected from drylaid, airlaid, wetlaid and spunbonded nonwovens and further has ii) a basis weight, determined as per DIN EN 29073-1, of 10 to 70 g/m² and iii) an absorbency for the alcoholic preparation of not less than 450% (wt./wt).

The nonwoven employed according to the invention is thus not a meltblown nonwoven. In one preferred embodiment, therefore, the carrier material does not comprise any meltblown nonwoven.

Preferably, the drylaid nonwoven is a carded nonwoven. It is further preferable for the nonwoven to be a spunlace nonwoven. In one preferred embodiment, the carrier material a) has a basis weight of 15 to 60 g/m², preferably 18 to 55 g/m², particularly 20 to 50 g/m², all determined as per DIN EN 29073-1.

In a further preferred nonwoven, the carrier material a) has an absorbency of not less than 500%, preferably not less than 550%, particularly not less than 600%, all (weight/weight).

The degree of impregnation of the impregnated textile fabric according to the invention is preferably in the range from 15 to 75%, more preferably 20 to 70%, particularly 25 to 70%, such as 30 to 70%, for example 35 to 65%.

Carrier materials comprise one or more polymers selected from polyethylene terephthalate (PET), polyolefin, viscose, cellulose and polylactic acid (PLA). Preferably, the polyolefin is polypropylene (PP).

In all embodiments of the invention, the carrier material a) may comprise blend fibres, in which case the blend fibres preferably comprise one or more of viscose/PP and viscose/PET.

According to the invention, the carrier material a) preferably consists essentially of polyolefin, particularly polypropylene. The carrier material a) more preferably consists of polyolefin, particularly polypropylene.

Carrier material a) aside, the impregnated textile fabrics according to the invention comprise the alcoholic preparation b) .

### b) Alcoholic preparation

The alcoholic preparation b) employed according to the invention comprises b1) two or more aliphatic C₂ to C₃ alcohols.

The alcoholic preparation b) is preferably a single-phase preparation. Alcoholic preparations in the form of a solution, a gel or a foam/mousse are particularly preferable.

The total amount of alcohol component b1) is preferably in the range from 30 to 80 wt%, more preferably 40 to 75 wt%, particularly 50 to 70 wt%, such as 55 to 65 wt%, all based on the weight of alcoholic preparation b).

Component b1) is a mixture of two aliphatic C₂ to C₃ alcohols. The following b1) components are particularly preferable:
- a mixture of ethanol and 1-propanol, and
- a mixture of 1-propanol and 2-propanol.

Component b1) aside, the alcoholic preparation employed in a preferred embodiment of the invention comprises one or more of the following optional components:
b2) one or more antimicrobial actives,
b3) one or more solvents, and/or
b4) one or more excipients.

Exemplary antimicrobial actives b2) are described in sections 39 to 62 of Wallhäußer's Praxis der Sterilisation, Desinfektion, Antiseptik und Konservierung (eds. A. Kramer and O. Assidian, Georg Thieme-Verlag Stuttgart, New York, 2008). They include aldehydes (such as formaldehyde, glutaraldehyde and succinaldehyde) and aldehyde depot compounds, anorganic acids, their salts and anhydrides, organic acids of carbon (such as aliphatic or aromatic carboxylic acids, their esters (such as parabens) and amide, oxidants (such as, for example, peroxides, particularly hydrogen peroxide and peracetic acid), phenol derivatives, surfactants (such as, in particular, quaternary ammonium salts and bispyridinioalkanes), guanidines and biguanides, alkylamines, nitrogen heterocycles, urea derivatives, aromatic alcohols, acetals and aminals, benzamidines, isothiazolines (such as isothiazolin-3-ones), phthalimide derivatives, quinolinols, benzimidazoles, mercaptobenzothiazoles, nitriles, carbamates (such as 3-iodo-2-propynyl butylcarbamate, IPBC), metals and metal compounds. Accordingly, component b2) may comprise a mixture of surfactants of the type which is typically used for cleaning, or consist of such a mixture of surfactants.

By way of surface-active ingredient b2), the alcoholic preparations according to the invention may comprise cationic, amphoteric and/or nonionic surfactants.

As non-ionic surfactant there may be employed any suitable nonionic surfactant, for example (i) (fatty) alcohol ethoxylates, (ii) sorbitan esters, (iii) alkylglycosides (particularly alkylpolyglucosides), (iv) ethylene oxide/- propylene oxide block copolymers. Alkylpolyglucosides (iii) are particularly preferred nonionic surfactants.

Alcohol polyalkoxylates (i) include fatty alcohol alkoxylates, for example isodecyl ethoxylates having various fractions of ethylene oxide, isotridecyl ethoxylates, polyethylene glycol ethers of stearyl, lauryl, cetyl and oleyl alcohols. The alcohols may have been alkoxylated with ethylene oxide, propylene oxide or any desired mixtures of ethylene oxide and propylene oxide. Alcohol polyalkoxylates are known, inter alia under the designations Lutensol^{®}, Marlipal^{®}, Marlox^{®}, Brij^{®} and Plurafac^{®}. Lauryl alcohol ethoxylates are particularly preferable for use as nonionic surfactant.

Useful nonionic surfactants (ii) further include sorbitan esters, which are usually in the form of oleates, stearates, laurates and palmitates and are referred to as polysorbates (e.g. Tween^{®}).

The nonionic surfactant may further be an alkylglycoside (iii), such as an alkylglucoside (i.e. an alkylglycoside of glucose), more preferably a C₈ to C₂₀ alkylpolyglucose, particularly a C₈ to C₁₆ alkylpolyglucose of a fatty alcohol, in which case a laurylpolyglucose, a decylpolyglucose or a mixture thereof is preferable. The carbon chain length is from 8 to 16 atoms in the case of cocoylpolyglucose, from 12 to 16 carbon atoms in the case of laurylpolyglucose and likewise from 8 to 16 carbon atoms in the case of decylpolyglucose.

Useful surfactants further include amphoteric surfactants, for example betaines. Suitable betaines are described in EP 0 560 114 A. Cocamidopropylbetaine is particularly preferable. N- Alkylaminopropylglycine is a further preferred amphoteric surfactant.

Useful surfactants further include cationic surfactants, such as quaternary ammonium salts. Any suitable quaternary ammonium compound is in principle useful in the present invention. The quaternary ammonium compound is preferably a dialkyldimethylammonium salt.

Quaternary ammonium salts preferred for use in the present invention are represented by the formula [R¹R²R³(CH₃)N]⁺[X]⁻, where R¹ to R³ may be the same or different and are selected from C₁ to C₃₀ alkyl, aralkyl, alkenyl and mixed groups, which may have one or more atoms selected from O, S, N and P, wherein R¹ to R³ are, for example, C₈ to C₁₈ alkyl, benzyl or methyl, preferably C₉ to C₁₈ alkyl, benzyl or methyl, such as C₁₆ alkyl, benzyl or methyl. X is an anion (of an organic acid or of an inorganic acid). This anion as well as the cation of the quaternary ammonium salt may each be a polyvalent ion, resulting in a [A⁽ⁿ⁺⁾]ₘ[K(^{m+)}]ₙ stoichiometry.

Useful quaternary ammonium salts for the purposes of the present invention include any prior art quaternary ammonium salts of the abovementioned formula, examples of which are disclosed in WO 00/63337 A, hereby incorporated herein by reference. Preferably, however, it is dialkyldimethylammonium salts which are used, for example dialkyldimethylammonium chlorides whose alkyl chains are each independently selected from C₈ to C₁₈ alkyl, preferably Cg to C₁₈ alkyl, such as C₁₆ alkyl. One of the methyl groups in the dialkyldimethylammonium salt may be an alkoxylated, for example ethoxylated, hydroxymethyl group.

Preferred quaternary ammonium salts for the purposes of the present invention are compounds of the formulae [R¹N(CH₃)₃]⁺[X]⁻, [R¹R²N(CH₃)₂]⁺[X]⁻ and [R¹R²R³(CH₃)N]⁺[X]⁻, where R¹ to R³ are each independently selected from C₈ to C₁₈ alkyl and -(CH₂-CHR⁴O)ₙ-R⁵, where n is from 1 to 20, preferably from 1 to 5, and R⁴ and R⁵, which may be the same or different, are each H and/or C₁ to C₄ alkyl, preferably H.

Exemplary anions and classes of anions for quaternary ammonium salts used according to the present invention are hydroxide, sulphate, hydrogensulphate, methosulphate, ethosulphate, laurylsulphate, lauryl ether sulphate, cellulosesulphate, sulphamate, halide (fluoride, chloride, bromide, iodide), nitrite, nitrate, carbonate, bicarbonate, phosphate, alkylphosphate, metaphosphate, polyphosphate, thiocyanate (rhodanide), carboxylic acid salt such as benzoate, lactate, acetate, propionate, citrate, succinate, glutarate, adipate, toluenesulphonate (tosylate) and salicylate. Chloride and propionate are particularly preferred anions.

Particular preference is given to using the quaternary ammonium salts mecetronium etilsulfate (hexadecyl(ethyl)dimethylammonium ethylsulphate), benzalkonium chloride and didecyldimethylammonium chloride.

The actives b2) preferred for a particularly high disinfecting effect include (list 1):
1) guanidines (preferably biguanidines),
2) aldehydes (such as, for example, formaldehyde, glutaraldehyde and succinaldehyde),
3) formaldehyde depot compounds, i.e. compounds that gradually release chemically bound formaldehyde in use,
4) active oxygen compounds such as peroxide compounds, peracetic acid and hydrogen peroxide,
5) alkylamines, and
6) amphosurfactants.

Actives b2) of primary interest for their cleaning effect are (list 2):
1) nonionic surfactants,
2) organic acids,
3) oxidizing agents, and
4) reducing agents.

Actives selected from the above lists 1 and 2 may additionally be included for their preservative effect.

### b3) Solvents

The alcoholic preparation used optionally further comprises b3) solvents. Preferred solvents are glycols and water and also mixtures thereof. Water is a preferred b3) component in all embodiments of the invention.

### b4) Excipients

Exemplary excipients for optional inclusion in the alcoholic preparations used according to the invention are perfumes, fragrances, thickeners, buffers, humectants, dyes, complexing agents, solubilizers, corrosion inhibitors and abrasives. Preferred pH values for the alcoholic preparations used according to the invention range from 1.0 to 12.0, more preferably from 1.5 to 11.5, as from 2.0 to 11.0.

Exemplary alcoholic preparations b) will now be described (alcoholic preparations 1 to 4):

### Alcoholic preparation 1

A preferred alcoholic preparation 1 has the following composition (Table 1a, particulars reported in wt%):

**Table la**

| **Constituent** | | **preferably at** | **more preferably at** | **particularly at** |
|---|---|---|---|---|
| b1 | ethanol | 10-50 | 15-40 | 20-30 |
| | 1-propanol | 20-60 | 25-50 | 30-40 |
| b2 | surfactant (particularly a nonionic surfactant) | 0.01-0.2 | 0.02-0.1 | 0.03-0.07 |
| b3 | water | balance | balance | Balance |
| b4 | perfume | 0.005-0.1 | 0.008-0.07 | 0.01-0.05 |

### Alcoholic preparation 2

A comparative alcoholic preparation 2 has the following composition (Table 1b, particulars reported in wt%):

**Table 1b**

| **Constituent** | | **preferably at** | **more preferably at** | **particularly at** |
|---|---|---|---|---|
| b1 | ethanol | 30-80 | 50-70 | 55-65 |
| b2 | surfactant (particularly an amphoteric surfactant) | 0.005-0.1 | 0.01-0.08 | 0.02-0.04 |
| b3 | water | balance | balance | Balance |
| b4 | perfume | 0.002-0.05 | 0.004-0.02 | 0.006-0.01 |

### Alcoholic preparation 3

A preferred alcoholic preparation 3 has the following composition (Table 1c, particulars reported in wt%).

**Table 1c**

| **Constituent** | | **preferably at** | **more preferably at** | **particularly at** |
|---|---|---|---|---|
| b1 | 2-propanol | 10-50 | 15-45 | 20-40 |
| | 1-propanol | 10-50 | 15-45 | 20-40 |
| b2 | organic acid (particularly benzoic acid, lactic acid or a mixture thereof) | 0.3-4 | 0.6-3 | 1-2 |
| b3 | water | balance | balance | Balance |
| b4 | base (pH adjustment) | 0.05-1 | 0.1-0.5 | 0.15-0.35 |
| | perfume | 0.03-0.3 | 0.06-0.2 | 0.08-0.15 |

A comparative alcoholic preparation 4 is an aqueous 2-propanol solution having a 2-propanol content of 40 to 80 wt%, preferably 50 to 75 wt%, more preferably 60 to 70 wt%.

The constituents reported for alcoholic preparations 1 to 4 are the constituents which are typically present. Further constituents may be present in these preparations.

Alternatively, the textile fabric may be provided from a) a carrier material and b) an alcoholic preparation in the form of a kit. This embodiment is preferable when, for example, the preparation comprises constituents which on prolonged contact with carrier material a) are not compatible therewith, as may possibly be the case with peroxide actives.

In a second aspect, therefore, the present invention provides a kit for the manufacture of textile fabrics impregnated with an alcoholic preparation, said kit comprising a) a carrier material and b) the alcoholic preparation,
wherein the carrier material
   i) comprises one or more nonwovens selected from drylaid, airlaid, wetlaid and spunbonded nonwovens,
   ii) has a basis weight, determined as per DIN EN 29073-1, of 10 to 70 g/m², and
   iii) has an absorbency for the alcoholic preparation of not less than 450% (wt./wt.),
wherein the amount of the alcoholic preparation b) is such that the percentage impregnation of the carrier material is not more than 80%,
and wherein the alcoholic preparation comprises b1) one or more aliphatic C₂- to C₃ alcohols, wherein the total amount of alcohol component b1) is in the range from 20 to 80 wt%, based on the weight of alcoholic preparation b).

In a third aspect, the present invention provides for the use of the impregnated fabric or of the kit in the disinfection of animate surfaces or inanimate surfaces.

In a fourth aspect, the present invention provides a process for disinfecting animate or inanimate surfaces, wherein the surface is subjected to the action of the impregnated fabric.

The present invention additionally provides a process for disinfecting and cleaning inanimate surfaces (hard surfaces in particular) with the impregnated textile fabric or the kit.

The advantages of the present invention are apparent from the following examples in particular. Percentages unless otherwise stated are by weight (wt%).

### Examples

### Method 1 - Determination of absorbency for the alcoholic preparation

The absorbency of the carrier material for the alcoholic preparation is determined in accordance with DIN 53923-1. To this end, in each case, a piece of the carrier material (10 x 10 cm) is weighed and secured with 4 paperclips to a metal grid (13.5 x 13.5 cm). The metal grid plus carrier material is immersed in 1 litre of the alcoholic preparation. After 1 min the grid plus the impregnated carrier material is removed and 3 of the paperclips are taken off. The impregnated carrier material is then inclined relative to the horizontal so as to hang freely and is let drip for 2 min. Thereafter, the impregnated carrier material is reweighed.

### Method 2 - Antimicrobial efficacy in the four-field test of EN 16615:2015

*Enterococcus hirae* was chosen as the challenge organism and the experiments were performed under clean conditions (0.03% bovine serum albumin), unless otherwise stated. The treatment time was 5 min. Different volumes of the test substance 70% (v/v) 2-propanol were applied to the standard test cloth defined in EN 16615:2015 (Tork cloth) at 8 ml, 16 ml or 24 ml, and the test was carried out as described in EN 16615:2015.

### Method 3 - Delivery characteristics

The delivered quantity is simulated with the four-field test of Method 2. To this end, the four-field test was carried out without admixture of challenge organisms to determine the delivered quantity.

Since it is not unambiguously quantifiable how much product is actually applied to the area (the rest ending up on the block, the hands, the Petri dish, etc.), the experimental set-up was supplemented. What is described hereinbelow is the EN 16615:2015 test as modified to demonstrate the advantages of the invention. The data obtained are more meaningful as a result of this modification:
- The size of the web was conformed to the standard block (a carrier material 7 x 11 cm in size is tested for good reproducibility).
- The nonwoven is impregnated on the block.
- The standard wiping procedure is carried out using the block.
- The delivery determined corresponds to the quantity applied to the area.

The standard block is provided the specific carrier material tested and Parafilm and placed on the scales. The alcoholic preparation is then applied until the defined amount of preparation is reached. Thereafter, the definition of the area is simulated in line with the four-field test. The block plus the carrier material (and alcoholic preparation remaining thereon) is then reweighed and the difference from the original overall weight is used to compute the preparation quantity applied to the test area.

### Example 1 - Influence of evaporation, not representative of the invention

Procedure:
The efficacy of 70% (v/v) 2-propanol was evaluated in accordance with DGHM methodology (Gebel, J., Werner, H.-P., Kirsch-Altena, A., Bansemir, K. (2001) : [in German] Standard Methods of the German Society for Hygiene and Microbiology (DGHM) for Testing Chemical Disinfection Procedures, Method 14: Area Disinfection - A Realistic Test, pp. 40-44, mhp Verlag Wiesbaden).

PVC test areas were evaluated in addition to the OP tiles. These PVC test areas were cut out in a size 5 x 5 cm from the test area DLW - Solid Pur 521 - 029 (as obtained from VAH). The test areas (OP tiles and PVC) were each contaminated with 50 µl of bacterial suspension (*Staphylococcus aureus* or *Enterococcus hirae*) under clean conditions (0.03% bovine serum albumin) and dried in ambient air for max. 60 min.

After drying the inoculums, the test areas were each disinfected with 3 different volumes (50, 100 or 200 µl each) of 70% (v/v) 2-propanol. The applied disinfectant was distributed with a spatula as per DGHM Method 14.1.5 a Area Disinfection - A Realistic Test of Wipe Disinfection. To evaluate the influence of the evaporation of the alcohol on microbiological efficacy, one test area (PVC or OP tile) per applied volume (50, 100 or 200 µl of 2-propanol) was evaluated at room temperature without cover. In addition, for comparison, one test area in each case was stored under identical conditions in a sealed Petri dish in order to minimize the flash-off effect of the 2-propanol.

The particular drying times were observed and recorded. After the treatment time of 5 min, the microorganisms were recovered from the test areas by means of glass beads on a horizontal shaker (DGHM Method No. 14.1.6 a). The data were analyzed as per DGHM Method 14.1.8. The lg RF values are depicted as means of three experiments (Fig. 1 and Fig. 2).

### Example 2: 2-Propanol

(Method 2, *E. hirae,* clean conditions, 0.03% BSA), not representative of the invention Table 2 shows that the application of increasing amounts of 2-propanol (70%, v/v) has an advantageous effect on the efficacy obtained in the course of a treatment time of 5 min:

**Table 2**

| | | | |
|---|---|---|---|
| | 8 ml | 16 ml | 24 ml |
| test field 1, lg | 3.15 | 7.58 | 7.42 |
| test fields 2-4, CFU/25 cm² | 0 | 0 | 0 |
| drying time, sec | 23 | 70 | 225 |
| solution transferred | 1.1 | 1.65 | 2.92 |

An amount of 8 ml does not lead to adequate microbicial efficacy in relation to the test organism in the EN 16615:2015 test, whereas an impregnation volume of 16 ml or 24 ml on the standard cloth used for disinfection leads to adequate efficacy as per EN 16615:2015 (RF ≥ 5 on test field 1; Ø ≤ 50 CFU / 25 cm² on test fields 2-4).

### Example 3 (Method 3)

Nineteen (19) nonwovens were evaluated under controlled environmental conditions (22°C, about 25% relative humidity) in order to capture the influence of grammage, raw material, fibre production, consolidation and finish. To this end, the particular carrier material was tested as described in Method 3 in combination with an alcoholic preparation (consisting of about 25 wt% of ethanol, 35 wt% of 1-propanol, 0.05 wt% of nonionic surfactant, 0.02 wt% of perfume, balance water), in that the particular carrier material was impregnated to 50%, 75% and 100%, all based on the maximum absorbency. All the values were determined in 3-plicate. Table 3 reports the data for i) maximum uptake and ii) delivery/uptake in terms of the respective means. Reproducibility was very good, in that the deviations from the mean were merely 8.1% in the maximum uptake test (i.e. as per Method 1) and 14.9% in the test for specific delivery.

**Table 3**

| | | | | | | | **50%²** | **75%²** | **100%²** |
|---|---|---|---|---|---|---|---|---|---|
| | | | | | **Thickness** | **Max. uptake¹** | **Delivery/uptake** | | |
| | **Grammage** | **Material** | **Technology** | **Consolidation** | **[mm]** | **[%]** | **[%]** | **[%]** | **[%]** |
| 1 | 20 g/m² | PP | spinbonding | spunlace | 0.327 | 769 | 56.9 | 52.7 | 47.5 |
| 2 | 40 g/m² | PP | spinbonding | spunlace | 0.455 | 553 | 42.1 | 36.0 | 33.8 |
| 3* | 75 g/m² | PP | spinbonding | spunlace | 0.570 | 458 | 26.2 | 22.7 | 24.4 |
| 4* | 75 g/m² | PP | spinbonding | spunlace | 0.525 | 350 | 21.5 | 23.8 | 26.1 |
| 5 | 24 g/m² | PP | carding | | 0.467 | 947 | 50.9 | 44.1 | 39.4 |
| 6 | 40 g/m² | PP | carding | | 0.460 | 557 | 40.2 | 36.5 | 35.0 |
| 7* | 75 g/m² | cellulose/PET | drylaid | | 0.447 | 456 | 18.1 | 21.9 | 23.0 |
| 8 | 40 g/m² | PET | drylaid | spunlace | 0.620 | 893 | 39.7 | 29.2 | 29.5 |
| 9 | 26 g/m² | PET | drylaid | spunlace | 0.600 | 1212 | 48.3 | 39.7 | 35.3 |
| 10* | 40 g/m² | PP | meltblown | | 0.360 | 473 | 17.3 | 19.5 | 26.1 |
| 11 | 48 g/m² | PET | drylaid | spunlace | 0.528 | 701 | 32.4 | 28.6 | 27.4 |
| 12* | 22 g/m² | cellulose/PET | wetlaid | | 0.102 | 362 | 36.3 | 36.0 | 37.9 |
| 13 | 19 g/m² | PP | spinbonding | | 0.200 | 537 | 53.2 | 47.5 | 39.2 |
| 14 | 20 g/m² | cellulose/PET | drylaid | spunbond | 0.120 | 497 | 40.2 | 42.7 | 41.6 |
| 16 | 50 g/m² | lyocell fibre | Tencel | | 0.565 | 840 | 25.9 | 22.8 | 23.0 |
| 17* | 80 g/m² | lyocell fibre | Tencel | | 2.957 | 1041 | 28.4 | 21.0 | 15.8 |
| 18 | 20 g/m² | PP | drylaid | spunbond | 0.287 | 600 | 51.6 | 47.4 | 58.3 |
| 19* | 20 g/m² | PP | drylaid | spunbond | 0.190 | 449 | 53.7 | 51.2 | 49.0 |

| | | | | | | | | | |
|---|---|---|---|---|---|---|---|---|---|
| * = comparison ¹ wt/wt ² degree of impregnation | | | | | | | | | |

The data in Table 3 show clearly that, for example, PP spunbondeds with spunlace consolidation even in the case of a high basis weight, a high maximum uptake (a high absorbency) and a high degree of impregnation do not necessarily lead to a high delivery (Comparative Examples 3 and 4). Delivery is very good, by contrast, in the case of a lower basis weight and high absorbency (Inventive Examples 1, 2 and 13). Good results regarding delivery are also achieved with carded PP webs (Inventive Examples 5 and 6). This also holds for Inventive Example 18 involving a drylaid PP web with spunbond consolidation.

Example 7, featuring a high basis weight for the carrier material, is for comparison and shows inadequate delivery, even at a 100% degree of impregnation. Examples 8, 9 and 11 evidence that even PET webs are employable for the purposes of the invention. Example 10 is for comparison and shows that meltblown webs do not evince the effect exploited according to the invention. Example 12 (cellulose/PET) is for comparison and shows that when absorbency is low, even a high degree of impregnation does not lead to high delivery, while Example 14 (likewise cellulose/PET) is in accordance with the present invention and evidences, by contrast, the advantages of high absorbency.

Examples 16 and 17, featuring a lyocell fibre, show that with these fibres (which have a high level of dry strength, wet strength and softness), delivery is inadequate even at a high degree of impregnation, on employing a web having a high grammage of 80 g/m² (even when coupled with a very high absorbency of 1041%, Comparative Example 17). Using by contrast, in accordance with the present invention, a web having a lower grammage of just 40 g/m², then delivery is adequate across a wide range of degrees of impregnation (Example 16).

**Example 4** - Possible ways to optimize the efficiency of cloth systems (cloth size 200 x 270 mm)

According to the invention, the delivery of alcoholic preparations out of impregnated textile fabrics can be made more effective:

**Table 4**

| | Comparison (cellulose/PET, wetlaid, chemical bonding) | Invention (PP) |
|---|---|---|
| grammage, g/m² | 22 | 20 |
| cloth size, mm | 200 x 270 | 200 x 270 |
| number of cloths per roll, pieces off | 200 | 200 |
| weight of nonwoven roll, g | 237.6 | 216 |
| impregnation quantity per pack unit, g | 680 | 680 |
| max. uptake, % | 362 | 769 |
| max. uptake, g | 860.1 | 1661 |
| impregnation quantity per pack unit, g | 680 | 680 |
| degree of impregnation, % | 79.1 | 40.9 |
| delivery/uptake, % | 37 | 59 |
| delivered quantity, g | 251 | 400 |

The data in Table 4 show that the invention achieves the higher delivered quantity with a carrier material of high maximum uptake capacity because - for the same quantity of impregnation - the degree of impregnation is lower in the invention, so the relative delivery, i.e. the delivery/uptake ratio, in %, is distinctly higher in the invention than in the comparative test.

## Claims

1. Textile fabric impregnated with an alcoholic preparation, said fabric comprising
a) a carrier material, and
b) the alcoholic preparation,
wherein
- the carrier material
i) comprises one or more nonwovens selected from drylaid, airlaid, wetlaid and spunbonded nonwovens,
ii) has a basis weight, determined as per DIN EN 29073-1, of 10 to 70 g/m² ,
iii) has an absorbency for the alcoholic preparation, determined in accordance with DIN 53923-1 of not less than 450% (wt./wt.), and
iv) comprises one or more polymers selected from polyethylene terephthalate (PET), polyolefin, viscose, cellulose and polylactic acid (PLA),
- the percentage impregnation of the carrier material is not more than 80%,
and wherein
- the alcoholic preparation comprises b1) two or more aliphatic C₂ - to C₃ alcohols, wherein the total amount of alcohol component b1) is in the range from 20 to 80 wt%, based on the weight of alcoholic preparation b).

2. Impregnated fabric according to Claim 1, **characterized in that** the drylaid nonwoven is a carded nonwoven.

3. Impregnated fabric according to Claim 1 or 2, **characterized in that** the nonwoven is a spunlace nonwoven.

4. Impregnated fabric according to any preceding claim, **characterized in that** the carrier material a) has a basis weight of 15 to 60 g/m², preferably 18 to 55 g/m², particularly 20 to 50 g/m².

5. Impregnated fabric according to any preceding claim, **characterized in that** the carrier material a) has an absorbency of not less than 500%, preferably not less than 550%, particularly not less than 600% (all (weight/weight)).

6. Fabric according to any preceding claim, **characterized in that** the percentage impregnation of the carrier material is in the range from 15 to 75%, more preferably 20 to 70%, particularly 25 to 70%, such as 30 to 70%, for example 35 to 65 %.

7. Impregnated fabric according to any preceding claim, **characterized in that** the polyolefin is polypropylene (PP).

8. Impregnated fabric according to any preceding claim, **characterized in that** the carrier material a) comprises blend fibres,
wherein the blend fibres preferably comprise one or more of viscose/PP and viscose/PET.

9. Impregnated fabric according to any of Claims 1 to 7, **characterized in that** the carrier material a) consists of polyolefin, preferably polypropylene.

10. Impregnated fabric according to any preceding claim, **characterized in that** the total amount of alcohol component b1) is in the range from 30 to 80 wt%, preferably 40 to 75 wt%, more preferably 50 to 70 wt%, particularly 55 to 65 wt%, all based on the weight of alcoholic preparation b).

11. Impregnated fabric according to any preceding claim, **characterized in that** component b1) is a mixture of two aliphatic C₂ to C₃ alcohols.

12. Impregnated fabric according to any preceding claim, **characterized in that** the alcoholic preparation b) further comprises
b2) one or more antimicrobial actives,
b3) one or more solvents, and/or
b4) one or more excipients.

13. Kit for the manufacture of textile fabrics impregnated with an alcoholic preparation, said kit comprising
a) a carrier material, and
b) the alcoholic preparation,
wherein
- the carrier material
i) comprises one or more nonwovens selected from drylaid, airlaid, wetlaid and spunbonded nonwovens,
ii) has a basis weight, determined as per DIN EN 29073-1, of 10 to 70 g/m²,
iii) has an absorbency for the alcoholic preparation of not less than 450% (wt./wt.), and
iv) comprises one or more polymers selected from polyethylene terephthalate (PET), polyolefin, viscose, cellulose and polylactic acid (PLA),
- the amount of the alcoholic preparation b) is such that the percentage impregnation of the carrier material is not more than 80%, and wherein
- the alcoholic preparation comprises b1) two or more aliphatic C₂ - to C₃ alcohols, wherein the total amount of alcohol component b1) is in the range from 20 to 80 wt%, based on the weight of alcoholic preparation b).

14. Use of the impregnated fabric according to any of Claims 1 to 12 or of the kit according to Claim 13 in the disinfection of inanimate surfaces.

15. Process for disinfecting inanimate surfaces, wherein the surface is subjected to the action of the impregnated fabric according to any of Claims 1 to 12.

16. Impregnated fabric according to any of Claims 1 to 12 or kit according to Claim 13 for use in the desinfection of animate surfaces.

## Patentansprüche

1. Mit einer alkoholischen Zubereitung getränktes textiles Flächengebilde, wobei das Flächengebilde
a) ein Trägermaterial und
b) die alkoholische Zubereitung
umfasst
worin
- das Trägermaterial
i) einen oder mehrere Vliesstoffe ausgewählt aus trockengelegten (Drylaid-), luftgelegten (Airlaid-), nassgelegten (Wetlaid-) und Spinn-Vliesstoffen umfasst,
ii) ein Flächengewicht, bestimmt gemäß DIN EN 29073-1, von 10 bis 70 g/m² aufweist,
iii) ein Aufnahmevermögen für die alkoholische Zubereitung von nicht weniger als 450% (Gew./Gew.) aufweist, und
iv) ein oder mehrere Polymere, ausgewählt aus Polyethylenterephthalat (PET), Polyolefin, Viskose, Zellulose und Polymilchsäure (PLA), umfasst,
- die prozentuale Tränkung des Trägermaterials nicht mehr als 80% beträgt, und worin
- die alkoholische Zubereitung b1) zwei oder mehr aliphatische C₂- bis C₃-Alkohole umfasst, worin die Gesamtmenge der Alkoholkomponente b1) im Bereich von 20 bis 80 Gew.-%, bezogen auf das Gewicht der alkoholischen Zubereitung b), liegt.

2. Getränktes Flächengebilde nach Anspruch 1, **dadurch gekennzeichnet, dass** der trockengelegte (Drylaid-)Vliesstoff ein kardierter Vliesstoff ist.

3. Getränktes Flächengebilde nach Anspruch 1 oder 2, **dadurch gekennzeichnet, dass** der Vliesstoff ein spinngelegter (Spunlace-)Vliesstoff ist.

4. Getränktes Flächengebilde nach einem der vorhergehenden Ansprüche, **dadurch gekennzeichnet, dass** das Trägermaterial a) ein Flächengewicht von 15 bis 60 g/m², vorzugsweise 18 bis 55 g/m², insbesondere 20 bis 50 g/m² aufweist.

5. Getränktes Flächengebilde nach einem der vorhergehenden Ansprüche, **dadurch gekennzeichnet, dass** das Trägermaterial a) ein Aufnahmevermögen von nicht weniger als 500%, bevorzugt nicht weniger als 550%, insbesondere nicht weniger als 600% (jeweils (Gew./Gew.)) aufweist.

6. Flächengebilde nach einem der vorhergehenden Ansprüche, **dadurch gekennzeichnet, dass** die prozentuale Tränkung des Trägermaterials im Bereich von 15 bis 75%, bevorzugter 20 bis 70%, insbesondere 25 bis 70%, wie 30 bis 70%, beispielsweise 35 bis 65%, liegt.

7. Getränktes Flächengebilde nach einem der vorhergehenden Ansprüche, **dadurch gekennzeichnet, dass** das Polyolefin Polypropylen (PP) ist.

8. Getränktes Flächengebilde nach einem der vorhergehenden Ansprüche, **dadurch gekennzeichnet, dass** das Trägermaterial a) Mischfasern umfasst, wobei die Mischfasern vorzugsweise eines oder mehrere von Viskose/PP und Viskose/PET umfassen.

9. Getränktes Flächengebilde nach einem der Ansprüche 1 bis 7, **dadurch gekennzeichnet, dass** das Trägermaterial a) aus Polyolefin besteht, vorzugsweise aus Polypropylen.

10. Getränktes Flächengebilde nach einem der vorhergehenden Ansprüche, **dadurch gekennzeichnet, dass** die Gesamtmenge der Alkoholkomponente b1) im Bereich von 30 bis 80 Gew.-%, vorzugsweise 40 bis 75 Gew.-%, bevorzugter 50 bis 70 Gew.-%, insbesondere 55 bis 65 Gew.-%, jeweils bezogen auf das Gewicht der alkoholischen Zubereitung b), liegt.

11. Getränktes Flächengebilde nach einem der vorhergehenden Ansprüche, **dadurch gekennzeichnet, dass** Komponente b1) ein Gemisch von zwei aliphatischen C₂- bis C₃-Alkoholen ist.

12. Getränktes Flächengebilde nach einem der vorhergehenden Ansprüche, **dadurch gekennzeichnet, dass** die alkoholische Zubereitung b) ferner
b2) ein oder mehrere antimikrobielle Wirkstoffe,
b3) ein oder mehrere Lösungsmittel und/oder
b4) einen oder mehrere Hilfsstoffe
umfasst.

13. Kit für die Herstellung von mit einer alkoholischen Zubereitung getränkten textilen Flächengebilden, wobei das Kit
a) ein Trägermaterial und
b) die alkoholische Zubereitung
umfasst,
worin
- das Trägermaterial
i) einen oder mehrere Vliesstoffe ausgewählt aus trockengelegten (Drylaid-), luftgelegten (Airlaid-), nassgelegten (Wetlaid-) und Spinn-Vliesstoffen umfasst,
ii) ein Flächengewicht, bestimmt gemäß DIN EN 29073-1, von 10 bis 70 g/m² aufweist,
iii) ein Aufnahmevermögen für die alkoholische Zubereitung von nicht weniger als 450% (Gew./Gew.) aufweist, und
iv) ein oder mehrere Polymere, ausgewählt aus Polyethylenterephthalat (PET), Polyolefin, Viskose, Zellulose und Polymilchsäure (PLA), umfasst,
- die Menge der alkoholischen Zubereitung b) so ist, dass die prozentuale Tränkung des Trägermaterials nicht mehr als 80% beträgt,
und worin
- die alkoholische Zubereitung b1) zwei oder mehr aliphatische C₂- bis C₃-Alkohole umfasst, worin die Gesamtmenge der Alkoholkomponente b1) im Bereich von 20 bis 80 Gew.-%, bezogen auf das Gewicht der alkoholischen Zubereitung b), liegt.

14. Verwendung des getränkten Flächengebildes gemäß einem der Ansprüche 1 bis 12 oder des Kits gemäß Anspruch 13 bei der Desinfektion von belebten Oberflächen oder unbelebten Oberflächen.

15. Verfahren zur Desinfektion belebter oder unbelebter Oberflächen, bei dem das getränkte Flächengebilde gemäß einem der Ansprüche 1 bis 12 auf die Oberfläche einwirken gelassen wird.

16. Getränktes Flächengebilde gemäß einem der Ansprüche 1 bis 12 oder Kit gemäß Anspruch 13 zur Verwendung in der Desinfektion von belebten Oberflächen.

## Revendications

1. Tissu textile imprégné d'une préparation alcoolique, ledit tissu comprenant
a) un matériau de support, et
b) la préparation alcoolique,
dans lequel
- le matériau de support
i) comprend un ou plusieurs non-tissés sélectionnés parmi des non-tissés par voie sèche, par voie pneumatique, par voie humide et par filage direct,
ii) présente une masse surfacique, déterminée conformément à DIN EN 29073-1, de 10 à 70 g/m²,
iii) présente une absorbance pour la préparation alcoolique, déterminée conformément à DIN 53923-1 pas inférieure à 450 % (p/p), et
iv) comprend un ou plusieurs polymères sélectionnés parmi un téréphtalate de polyéthylène (PET), une polyoléfine, une viscose, une cellulose et un acide polylactique (PLA),
- le pourcentage d'imprégnation du matériau de support n'est pas supérieur à 80 %,
et dans lequel
- la préparation alcoolique comprend b1) deux alcools aliphatiques ou plus en C₂ à C₃, dans lequel la quantité totale du composant alcool b1) se trouve dans la plage de 20 à 80 % en poids, sur la base du poids de la préparation alcoolique b).

2. Tissu imprégné selon la revendication 1, **caractérisé en ce que** le non-tissé par voie sèche est un non-tissé cardé.

3. Tissu imprégné selon la revendication 1 ou 2, **caractérisé en ce que** le non-tissé est un non-tissé lacé par filage.

4. Tissu imprégné selon une quelconque revendication précédente, **caractérisé en ce que** le matériau de support a) présente une masse surfacique de 15 à 60 g/m², de préférence de 18 à 55 g/m², notamment de 20 à 50 g/m².

5. Tissu imprégné selon une quelconque revendication précédente, **caractérisé en ce que** le matériau de support a) présente une absorbance pas inférieure à 500 %, de préférence pas inférieure à 550 %, notamment pas inférieure à 600 % (tous en (p/p)).

6. Tissu selon une quelconque revendication précédente, **caractérisé en ce que** le pourcentage d'imprégnation du matériau de support se trouve dans la plage de 15 à 75 %, de manière davantage préférée de 20 à 70 %, notamment de 25 à 70 %, par exemple de 30 à 70 %, par exemple de 35 à 65 %.

7. Tissu imprégné selon une quelconque revendication précédente, **caractérisé en ce que** la polyoléfine est un polypropylène (PP).

8. Tissu imprégné selon une quelconque revendication précédente, **caractérisé en ce que** le matériau de support a) comprend des fibres mélangées,
dans lequel les fibres mélangées comprennent de préférence un ou plusieurs de viscose/PP et de viscose/PET.

9. Tissu imprégné selon l'une quelconque des revendications 1 à 7, **caractérisé en ce que** le matériau de support a) est constitué de polyoléfine, de préférence de polypropylène.

10. Tissu imprégné selon une quelconque revendication précédente, **caractérisé en ce que** la quantité totale de composant alcool b1) se trouve dans la plage de 30 à 80 % en poids, de préférence de 40 à 75 % en poids, de manière davantage préférée de 50 à 70 % en poids, notamment de 55 à 65 % en poids, tous sur la base du poids de la préparation alcoolique b).

11. Tissu imprégné selon une quelconque revendication précédente, **caractérisé en ce que** le composant b1) est un mélange de deux alcools aliphatiques en C₂ à C₃.

12. Tissu imprégné selon une quelconque revendication précédente, **caractérisé en ce que** la préparation alcoolique b) comprend en outre
b2) un ou plusieurs actifs antimicrobiens,
b3) un ou plusieurs solvants, et/ou
b4) un ou plusieurs excipients.

13. Kit de fabrication de tissus textiles imprégnés avec une préparation alcoolique, ledit kit comprenant
a) un matériau de support, et
b) la préparation alcoolique,
dans lequel
- le matériau de support
i) comprend un ou plusieurs non-tissés sélectionnés parmi des non-tissés par voie sèche, par voie pneumatique, par voie humide et par filage direct,
ii) présente une masse surfacique, déterminée conformément à DIN EN 29073-1, de 10 à 70 g/m²,
iii) présente une absorbance pour la préparation alcoolique pas inférieure à 450 % (p/p), et
iv) comprend un ou plusieurs polymères sélectionnés parmi un téréphtalate de polyéthylène (PET), une polyoléfine, une viscose, une cellulose et un acide polylactique (PLA),
- la quantité de préparation alcoolique b) est telle que le pourcentage d'imprégnation du matériau de support n'est pas supérieur à 80 %,
et dans lequel
- la préparation alcoolique comprend b1) deux alcools aliphatiques ou plus en C₂ à C₃, dans lequel la quantité totale du composant alcool b1) se trouve dans la plage de 20 à 80 % en poids, sur la base du poids de la préparation alcoolique b1).

14. Utilisation du tissu imprégné selon l'une quelconque des revendications 1 à 12 ou du kit selon la revendication 13 dans la désinfection de surfaces inanimées.

15. Procédé de désinfection de surface inanimées, dans lequel la surface est soumise à l'action du tissu imprégné selon l'une quelconque des revendications 1 à 12.

16. Tissu imprégné selon l'une quelconque des revendications 1 à 12 ou kit selon la revendication 13 pour son utilisation dans la désinfection de surfaces animées.
